# EUROPEAN PATENT APPLICATION

(11) **EP 4 717 688 A1**
(43) Date of publication of application: **01.04.2026**
(21) Application number: 24835979.6
(22) Date of filing: 27.06.2024
(51) Int. Cl.: C07C 45/59, B01J 23/44, C07C 45/80, C07C 49/395, C07B 61/00

(54) **PRODUCTION METHOD FOR CYCLOPENTANONE AND PURIFIED CYCLOPENTANONE**

(30) Priority: 05.07.2023 JP 2023110573
(71) Applicant: ENEOS CORPORATION, Chiyoda-ku Tokyo 100-8162 (JP)
(72) Inventor: UMEDA, Tadashi, Tokyo 100-8162 (JP); IWASA, Yasuyuki, Tokyo 100-8162 (JP); URUSHIZAKO, Naoko, Tokyo 100-8162 (JP); SATO, Koichi, Tokyo 100-8162 (JP)
(74) Representative: Grünecker Patent- und Rechtsanwälte PartG mbB
(86) International application number: PCT/JP2024/023399
(87) International publication number: WO 2025/009466

(57) **Abstract**

A production method for cyclopentanone including: a rearrangement hydrogenation step of passing a first fluid containing furfuryl alcohol, water, and hydrogen through a fixed-bed flow reactor packed with a hydrogenation catalyst to obtain a reaction product containing cyclopentanone and water; an extraction step of extracting cyclopentanone from the reaction product with an extraction solvent to obtain an oil phase containing cyclopentanone; and a recovery step of recovering cyclopentanone from the oil phase.

## Description

### Technical Field

The present disclosure relates to a production method for cyclopentanone and purified cyclopentanone.

### Background Art

Cyclopentanone is used for various purposes such as a raw material for producing jasmine-based perfumes, a raw material for producing lactone-based perfumes, and a semiconductor cleaning agent. Conventionally, known production methods for cyclopentanone include a production method involving a ketonization reaction of adipic acid and a production method involving a dehydrogenation reaction of cyclopentanol. For example, Patent Literature 1 describes a method for producing cyclopentanone by a vapor-phase dehydrogenation reaction of cyclopentanol in the presence of a specific catalyst.

### Citation List

### Patent Literature

[Patent Literature 1] Japanese Unexamined Patent Publication No. 2017-178824

### Summary of Invention

### Technical Problem

Conventional production methods for cyclopentanone have a problem in that they are production methods using petroleum-derived raw materials.

An object of the present disclosure is to provide a production method for cyclopentanone that enables efficient production of cyclopentanone using raw materials that are readily available from biomass. Another object of the present disclosure is to provide purified cyclopentanone.

### Solution to Problem

The present disclosure relates to, for example, the following [1] to [4].
[1] A production method for cyclopentanone including:
   a rearrangement hydrogenation step of bringing a first fluid containing furfuryl alcohol, water, and hydrogen into contact with a hydrogenation catalyst to obtain a reaction product containing cyclopentanone and water;
   an extraction step of extracting cyclopentanone from the reaction product with an extraction solvent to obtain an oil phase containing cyclopentanone; and
   a recovery step of recovering cyclopentanone from the oil phase.
[2] The production method according to [1], further including:
   a separation step of removing at least a part of the extraction solvent from the oil phase; and
   a purification step of purifying cyclopentanone from a residue of the separation step.
[3] The production method according to [1] or [2], in which the extraction solvent includes at least one selected from the group consisting of aromatic hydrocarbons and alicyclic hydrocarbons.
[4] Purified cyclopentanone including:
   cyclopentanone; and
   at least one trace component selected from the group consisting of furfural, 4-hydroxy-2-cyclopentenone, furfuryl alcohol, 2-cyclopentenone, toluene, ethylbenzene, ethylcyclohexane, and dimethylcyclohexane,
   wherein a content of the cyclopentanone is 98 mass% or more,
   a content of the trace component is 0.1 mass% or more and less than 2 mass%, and
   a percentage of 4-hydroxy-2-cyclopentenone and furfuryl alcohol in the trace component is less than 50 mass%.

### Advantageous Effects of Invention

According to the present disclosure, there is provided a production method for cyclopentanone that enables efficient production of cyclopentanone using raw materials that are readily available from biomass. According to the present disclosure, there is also provided purified cyclopentanone obtained by the production method.

### Brief Description of Drawings

FIG. 1 is a schematic diagram showing an example of a production apparatus for carrying out a production method of the present embodiment.
FIG. 2 is a schematic diagram showing a production apparatus of Comparative Example 1.
FIG. 3 is a schematic diagram showing another example of a production apparatus for carrying out the production method of the present embodiment.

### Description of Embodiment

Hereinafter, a suitable embodiment of the present disclosure will be described in detail.

A production method for cyclopentanone of the present embodiment includes: a rearrangement hydrogenation step of bringing a first fluid containing furfuryl alcohol, water, and hydrogen into contact with a hydrogenation catalyst to obtain a reaction product containing cyclopentanone and water; an extraction step of extracting cyclopentanone from the reaction product with an extraction solvent to obtain an oil phase containing cyclopentanone; and a recovery step of recovering cyclopentanone from the oil phase.

According to the production method of the present embodiment, cyclopentanone can be efficiently produced using furfuryl alcohol, which is readily available from biomass, as a raw material.

Each step of the production method of the present embodiment will be described in detail below.

### (Rearrangement hydrogenation step)

The rearrangement hydrogenation step is a step of bringing a first fluid containing furfuryl alcohol, water, and hydrogen into contact with a hydrogenation catalyst to obtain a reaction product containing cyclopentanone and water. In the rearrangement hydrogenation step, cyclopentanone is produced by a rearrangement hydrogenation reaction (rearrangement reaction and hydrogenation reaction) of furfuryl alcohol.

Furfuryl alcohol can be obtained, for example, by hydrogenating furfural obtained by hydrolysis of hemicellulose. In the present embodiment, the production method for furfuryl alcohol is not particularly limited and may be the above method or any other method.

The first fluid may contain: an oxygen-containing organic component including furfuryl alcohol; water; and hydrogen.

A percentage of furfuryl alcohol in the oxygen-containing organic component may be, for example, 50 mol% or more, and may be 80 mol% or more, 90 mol% or more, 95 mol% or more, 98 mol% or more, 99 mol% or more, or 99.5 mol% or more, and may be 100 mol%.

The oxygen-containing organic component is an organic component having an oxygen atom. The oxygen-containing organic component may further contain other components in addition to furfuryl alcohol. Examples of other components include products obtained by hydrolysis of hemicellulose (for example, furfural, formic acid, acetic acid, and hydroxymethylfurfural), by-products of a hydrogenation reaction of furfural (for example, tetrahydrofurfuryl alcohol, methylfuran, and methyltetrahydrofuran), and intermediates and by-products of a rearrangement hydrogenation reaction from furfuryl alcohol to cyclopentanone (for example, 4-hydroxy-2-cyclopentenone, 2-cyclopentenone, cyclopentanol, levulinic acid, and butanal).

A hydrogen-to-oil ratio (a molar ratio of hydrogen to the oxygen-containing organic component) in the first fluid may be, for example, 1 or more, and from the viewpoint of further promoting the hydrogenation reaction, may be 2 or more, 3 or more, or 4 or more. In addition, the hydrogen-to-oil ratio in the first fluid may be, for example, 50 or less, and from the viewpoint of further improving the selectivity in the rearrangement hydrogenation reaction, may be 40 or less, 30 or less, or 20 or less. That is, the hydrogen-to-oil ratio in the first fluid may be, for example, 1 to 50, 1 to 40, 1 to 30, 1 to 20, 2 to 50, 2 to 40, 2 to 30, 2 to 20, 3 to 50, 3 to 40, 3 to 30, 3 to 20, 4 to 50, 4 to 40, 4 to 30, or 4 to 20.

In the first fluid, a mass ratio (water/oxygen-containing organic component) of water to the oxygen-containing organic component may be, for example, 5 or more, and from the viewpoint of further suppressing polymerization of furfuryl alcohol, may be 7 or more, 10 or more, or 15 or more. In addition, the mass ratio (water/oxygen-containing organic component) of water to the oxygen-containing organic component may be, for example, 100 or less, and from the viewpoint of further improving the recovery efficiency of cyclopentanone, may be 70 or less, 50 or less, or 40 or less. That is, the mass ratio (water/oxygen-containing organic component) of water to the oxygen-containing organic component may be, for example, 5 to 100, 5 to 70, 5 to 50, 5 to 40, 7 to 100, 7 to 70, 7 to 50, 7 to 40, 10 to 100, 10 to 70, 10 to 50, 10 to 40, 15 to 100, 15 to 70, 15 to 50, or 15 to 40.

The first fluid may further contain other components in addition to the oxygen-containing organic component, water, and hydrogen. Examples of the other components include, for example, toluene and benzene. A percentage of the other components in the first fluid may be, for example, 5 volume% or less, and may be 3 volume% or less, 2 volume% or less, 1 volume% or less, or 0.5 volume% or less, and may be 0 volume%. That is, a total amount of the oxygen-containing organic component, water, and hydrogen in the first fluid may be, based on the total amount of the first fluid, for example, 95 volume% or more, and may be 97 volume% or more, 98 volume% or more, 99 volume% or more, or 99.5 volume% or more, and may be 100 volume%.

The hydrogenation catalyst is not particularly limited as long as it is a catalyst capable of converting at least a part of 4-hydroxy-2-cyclopentenone and 2-cyclopentenone into cyclopentanone. The hydrogenation catalyst may be appropriately selected from solid catalysts used in known hydrogenation reactions.

The hydrogenation catalyst may be, for example, a catalyst in which an active metal is supported on a carrier.

The carrier may be, for example, an inorganic oxide carrier. The inorganic oxide carrier may be, for example, an inorganic oxide such as silica, alumina, or titania.

Examples of the active metal include platinum and palladium.

In the hydrogenation catalyst, an amount of the active metal supported is not particularly limited. The amount of the active metal supported may be, based on the total amount of the hydrogenation catalyst, for example, 0.01 mass% or more, and may be 0.02 mass% or more, 0.025 mass% or more, or 0.03 mass% or more. In addition, the amount of the active metal supported may be, based on the total amount of the hydrogenation catalyst, for example, 1 mass% or less, and may be 0.5 mass% or less, 0.3 mass% or less, or 0.1 mass% or less. That is, the amount of the active metal supported may be, based on the total amount of the hydrogenation catalyst, for example, 0.01 to 1 mass%, 0.01 to 0.5 mass%, 0.01 to 0.3 mass%, 0.01 to 0.1 mass%, 0.02 to 1 mass%, 0.02 to 0.5 mass%, 0.02 to 0.3 mass%, 0.02 to 0.1 mass%, 0.025 to 1 mass%, 0.025 to 0.5 mass%, 0.025 to 0.3 mass%, 0.025 to 0.1 mass%, 0.03 to 1 mass%, 0.03 to 0.5 mass%, 0.03 to 0.3 mass%, 0.03 to 0.1 mass%, 0.1 to 1 mass%, 0.1 to 0.5 mass%, 0.1 to 0.3 mass%, or 0.1 to 0.1 mass%.

A reactor used in the rearrangement hydrogenation step is not particularly limited as long as it is a reactor that can be packed with a hydrogenation catalyst and brings the first fluid into contact with the packed hydrogenation catalyst. For example, a fixed-bed flow reactor is preferred. Examples of the fixed-bed flow reactor include a cylindrical reactor, a radial-flow reactor, a tubular reactor (single-tube reactor or multi-tube reactor), and a double-tube reactor.

A reaction temperature of the rearrangement hydrogenation reaction (for example, a temperature of a packed portion of the fixed-bed flow reactor in which the hydrogenation catalyst is packed) may be, for example, 150°C or more, and from the viewpoint of further improving the selectivity of the rearrangement reaction, may be 170°C or more, 180°C or more, or 190°C or more. In addition, the reaction temperature of the rearrangement hydrogenation reaction may be, for example, 250°C or less, and from the viewpoint of further suppressing polymerization of furfuryl alcohol, may be 230°C or less, 220°C or less, or 210°C or less. That is, the reaction temperature of the rearrangement hydrogenation reaction may be, for example, 150°C to 250°C, 150°C to 230°C, 150°C to 220°C, 150°C to 210°C, 170°C to 250°C, 170°C to 230°C, 170°C to 220°C, 170°C to 210°C, 180°C to 250°C, 180°C to 230°C, 180°C to 220°C, 180°C to 210°C, 190°C to 250°C, 190°C to 230°C, 190°C to 220°C, or 190°C to 210°C.

A reaction pressure of the rearrangement hydrogenation reaction (for example, a pressure of a packed portion of the fixed-bed flow reactor in which the hydrogenation catalyst is packed) may be, for example, 1.0 MPaG or more, and from the viewpoint of further promoting the hydrogenation reaction, may be 2.0 MPaG or more, 2.5 MPaG or more, or 3.0 MPaG or more. In addition, the reaction pressure of the rearrangement hydrogenation reaction (for example, the pressure of the packed portion of the fixed-bed flow reactor in which the hydrogenation catalyst is packed) may be, for example, 20.0 MPaG or less, and from the viewpoint of further improving the reaction selectivity, may be 15.0 MPaG or less, 10.0 MPaG or less, or 8.0 MPaG or less. That is, the reaction pressure of the rearrangement hydrogenation reaction may be, for example, 1.0 to 20.0 MPaG, 1.0 to 15.0 MPaG, 1.0 to 10.0 MPaG, 1.0 to 8.0 MPaG, 2.0 to 20.0 MPaG, 2.0 to 15.0 MPaG, 2.0 to 10.0 MPaG, 2.0 to 8.0 MPaG, 2.5 to 20.0 MPaG, 2.5 to 15.0 MPaG, 2.5 to 10.0 MPaG, 2.5 to 8.0 MPaG, 3.0 to 20.0 MPaG, 3.0 to 15.0 MPaG, 3.0 to 10.0 MPaG, or 3.0 to 8.0 MPaG.

A WHSV (a ratio of the weight of the oxygen-containing organic component supplied to the reactor per unit time to the weight of the catalyst packed in the reactor) in the rearrangement hydrogenation reaction may be, for example, 0.005 h⁻¹ or more, and from the viewpoint of further improving the reaction selectivity, may be 0.01 h⁻¹ or more, 0.015 h⁻¹ or more, or 0.02 h⁻¹ or more. In addition, the WHSV in the rearrangement hydrogenation reaction may be, for example, 1.0 h⁻¹ or less, and from the viewpoint of promoting the rearrangement hydrogenation reaction, may be 0.5 h⁻¹ or less, 0.2 h⁻¹ or less, or 0.1 h⁻¹ or less. That is, the WHSV in the rearrangement hydrogenation reaction may be, for example, 0.005 to 1.0 h⁻¹, 0.005 to 0.5 h⁻¹, 0.005 to 0.2 h⁻¹, 0.005 to 0.1 h⁻¹, 0.01 to 1.0 h⁻¹, 0.01 to 0.5 h⁻¹, 0.01 to 0.2 h⁻¹, 0.01 to 0.1 h⁻¹, 0.015 to 1.0 h⁻¹, 0.015 to 0.5 h⁻¹, 0.015 to 0.2 h⁻¹, 0.015 to 0.1 h⁻¹, 0.02 to 1.0 h⁻¹, 0.02 to 0.5 h⁻¹, 0.02 to 0.2 h⁻¹, or 0.02 to 0.1 h⁻¹.

In the rearrangement hydrogenation reaction, a conversion rate of furfuryl alcohol may be, for example, 70% or more, and from the viewpoint of further suppressing polymerization of furfuryl alcohol during recycling, may be 80% or more, 90% or more, 95% or more, or 98% or more. In addition, the conversion rate of furfuryl alcohol in the rearrangement hydrogenation reaction may be, for example, 100% or less, and from the viewpoint of improving reaction selectivity, may be 99.9% or less, 99.8% or less, or 99.5% or less. The rearrangement hydrogenation step may be appropriately adjusted so that the conversion rate falls within the above range. That is, the conversion rate of furfuryl alcohol in the rearrangement hydrogenation reaction may be, for example, 70% to 100%, 70% to 99.9%, 70% to 99.8%, 70% to 99.5%, 80% to 100%, 80% to 99.9%, 80% to 99.8%, 80% to 99.5%, 90% to 100%, 90% to 99.9%, 90% to 99.8%, 90% to 99.5%, 95% to 100%, 95% to 99.9%, 95% to 99.8%, 95% to 99.5%, 98% to 100%, 98% to 99.9%, 98% to 99.8%, or 98% to 99.5%.

In the rearrangement hydrogenation step, a reaction product containing cyclopentanone and water is obtained. The reaction product may be a liquid phase obtained by gas-liquid separation of a second fluid recovered from an outlet of the fixed-bed flow reactor, for example.

The reaction product may be a fluid (third fluid) containing water and an oxygen-containing organic component including cyclopentanone.

The reaction product may further contain an oxygen-containing organic component other than cyclopentanone. For example, the reaction product may contain components that were included in the first fluid (for example, furfuryl alcohol and water). The reaction product may also further contain intermediates and by-products of a rearrangement hydrogenation reaction from furfuryl alcohol to cyclopentanone (for example, 4-hydroxy-2-cyclopentanone, 2-cyclopentenone, cyclopentanol, levulinic acid, and butanal).

### (Extraction step)

The extraction step is a step of extracting cyclopentanone from a reaction product (third fluid) obtained in the rearrangement hydrogenation step with an extraction solvent to obtain an oil phase containing cyclopentanone. In the extraction step, from the reaction product containing a large amount of water, an oxygen-containing organic component including cyclopentanone is extracted by the extraction solvent, and water forming an azeotrope with cyclopentanone is separated as an aqueous phase. The extraction solvent may be used alone or in combination of two or more thereof.

The extraction solvent may be any solvent that separates from water and can extract cyclopentanone. Examples of the extraction solvent include aromatic hydrocarbons (for example, benzene, toluene, xylene, mesitylene, and 1,2,3,5-tetramethylbenzene) and alicyclic hydrocarbons (for example, cyclohexane, methylcyclohexane, cyclohexene, and methylcyclohexene).

The extraction solvent preferably includes at least one selected from the group consisting of aromatic hydrocarbons and alicyclic hydrocarbons. In addition, in the extraction step, it is preferable that 50 mass% or more (more preferably 70 mass% or more, still more preferably 90 mass% or more, and particularly preferably 100 mass%) of the extraction solvent is a solvent selected from the group consisting of aromatic hydrocarbons and alicyclic hydrocarbons, and the entire extraction solvent may be a solvent selected from the group consisting of aromatic hydrocarbons and alicyclic hydrocarbons. As the aromatic hydrocarbon, benzene and toluene are preferable. As the alicyclic hydrocarbons, cyclohexane and methylcyclohexane are preferable.

An amount of the extraction solvent used is not particularly limited as long as it is an amount sufficient to extract cyclopentanone (for example, an amount capable of extracting 90% or more (preferably 95% or more) of cyclopentanone in the reaction product).

The amount of the extraction solvent used may be, based on 100 parts by mass of the reaction product, for example, 20 parts by mass or more, and may be 30 parts by mass or more, 35 parts by mass or more, or 40 parts by mass or more. In addition, the amount of the extraction solvent used may be, based on 100 parts by mass of the reaction product, for example, 100 parts by mass or less, and may be 90 parts by mass or less, 80 parts by mass or less, or 70 parts by mass or less. That is, the amount of the extraction solvent used may be, based on 100 parts by mass of the reaction product, for example, 20 to 100 parts by mass, 20 to 90 parts by mass, 20 to 80 parts by mass, 20 to 70 parts by mass, 30 to 100 parts by mass, 30 to 90 parts by mass, 30 to 80 parts by mass, 30 to 70 parts by mass, 35 to 100 parts by mass, 35 to 90 parts by mass, 35 to 80 parts by mass, 35 to 70 parts by mass, 40 to 100 parts by mass, 40 to 90 parts by mass, 40 to 80 parts by mass, or 40 to 70 parts by mass.

An extraction temperature may be, for example, 20°C or more, and from the viewpoint of operational management efficiency, may be 30°C or more, 35°C or more, or 40°C or more. In addition, the extraction temperature may be, for example, 90°C or less, and from the viewpoint of recovery efficiency of the target component, may be 80°C or less, 70°C or less, or 60°C or less. That is, the extraction temperature may be, for example, 20°C to 90°C, 20°C to 80°C, 20°C to 70°C, 20°C to 60°C, 30°C to 90°C, 30°C to 80°C, 30°C to 70°C, 30°C to 60°C, 35°C to 90°C, 35°C to 80°C, 35°C to 70°C, 35°C to 60°C, 40°C to 90°C, 40°C to 80°C, 40°C to 70°C, or 40°C to 60°C.

The extraction step may be performed using a known extraction apparatus. Examples of the extraction apparatus include a mixer-settler type extraction apparatus and a column-type extraction apparatus. In the extraction step, these may be used alone or a plurality of extraction apparatuses may be used in multiple stages.

An aqueous phase separated in the extraction step may be treated as wastewater. An oil phase (fourth fluid) obtained in the extraction step is subjected to a recovery step described below.

### (Recovery step)

The recovery step is a step of recovering cyclopentanone (purified cyclopentanone) from the oil phase (fourth fluid) obtained in the extraction step.

The oil phase contains: at least a part of the oxygen-containing organic component contained in the reaction product; and an extraction solvent. The recovery step can also be regarded as a step of removing the extraction solvent and an oxygen-containing organic component other than cyclopentanone to obtain cyclopentanone (purified cyclopentanone).

The recovery step may include, for example, a separation step of removing at least a part of the extraction solvent from the oil phase, and a purification step of purifying cyclopentanone from a residue of the separation step.

In the separation step, a method for removing the extraction solvent is not particularly limited, and may be, for example, a method of removing the extraction solvent by utilizing a difference in boiling points between the extraction solvent and cyclopentanone. The separation step may be performed using a known distillation apparatus.

In the separation step, most of the extraction solvent may be removed. A residue of the separation step may contain the oxygen-containing organic component and may further contain a part of the extraction solvent. A content of the extraction solvent in the residue of the separation step may be, for example, 5.0 mass% or less, and may be 3.0 mass% or less, 2.0 mass% or less, or 1.0 mass% or less, and may be 0 mass%.

The extraction solvent removed in the separation step may be reused in the extraction step.

In the purification step, cyclopentanone (purified cyclopentanone) is obtained by removing at least a part of components other than cyclopentanone (the oxygen-containing organic component other than cyclopentanone; and the extraction solvent) from the residue of the separation step.

A purification method used in the purification step is not particularly limited, and examples thereof include purification by distillation utilizing a difference in boiling points and purification by membrane separation.

The purified cyclopentanone obtained in the recovery step may contain, for example, cyclopentanone and a trace component other than cyclopentanone.

In the purified cyclopentanone, a content of cyclopentanone may be, for example, 95 mass% or more, and preferably 98 mass% or more.

The trace component may be at least one selected from the group consisting of furfural, 4-hydroxy-2-cyclopentenone, furfuryl alcohol, 2-cyclopentenone, toluene, ethylbenzene, ethylcyclohexane, dimethylcyclohexane, and 1,2,3,5-tetramethylbenzene.

Among these, furfural, 4-hydroxy-2-cyclopentenone, furfuryl alcohol, and 2-cyclopentenone may be unreacted raw materials or by-products of the rearrangement hydrogenation reaction. In addition, toluene, ethylbenzene, ethylcyclohexane, dimethylcyclohexane, and 1,2,3,5-tetramethylbenzene may be extraction solvents or reaction products thereof in the extraction step.

A content of the trace component may be, for example, 0.1 mass% or more, 0.4 mass% or more, or 0.8 mass% or more. In addition, the content of the trace component may be, for example, less than 2 mass%, less than 1.5 mass%, or less than 1.2 mass%. That is, the content of the trace component may be, for example, 0.1 mass% or more and less than 2 mass%, 0.1 mass% or more and less than 1.5 mass%, 0.1 mass% or more and less than 1.2 mass%, 0.4 mass% or more and less than 2 mass%, 0.4 mass% or more and less than 1.5 mass%, 0.4 mass% or more and less than 1.2 mass%, 0.8 mass% or more and less than 2 mass%, 0.8 mass% or more and less than 1.5 mass%, or 0.8 mass% or more and less than 1.2 mass%.

A percentage of 4-hydroxy-2-cyclopentenone and furfuryl alcohol in the trace component may be, for example, less than 50 mass%, and preferably 40 mass% or less, more preferably 30 mass% or less. Such purified cyclopentanone has a low content of components having a hydroxyl group, and thus can sufficiently reduce side reactions caused by hydroxyl groups and is easily applicable to various reactions. A percentage of 4-hydroxy-2-cyclopentenone and furfuryl alcohol in the trace component may be, for example, 0 mass%, or may be 1 mass% or more, 5 mass% or more, 10 mass% or more, 15 mass% or more, or 20 mass% or more. That is, the percentage of 4-hydroxy-2-cyclopentenone and furfuryl alcohol in the trace component may be, for example, 0 mass% or more and less than 50 mass%, 0 mass% to 40 mass%, 0 mass% to 30 mass%, 1 mass% or more and less than 50 mass%, 1 mass% to 40 mass%, 1 mass% to 30 mass%, 5 mass% or more and less than 50 mass%, 5 mass% to 40 mass%, 5 mass% to 30 mass%, 10 mass% or more and less than 50 mass%, 10 mass% to 40 mass%, 10 mass% to 30 mass%, 15 mass% or more and less than 50 mass%, 15 mass% to 40 mass%, 15 mass% to 30 mass%, 20 mass% or more and less than 50 mass%, 20 mass% to 40 mass%, or 20 mass% to 30 mass%.

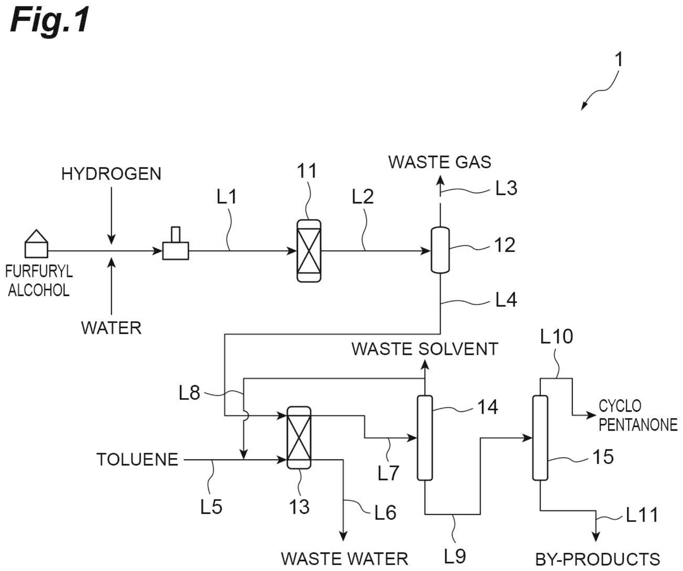

FIG. 1 is a schematic diagram showing an example of a production apparatus for carrying out a production method of the present embodiment.

A production apparatus 1 in FIG. 1 includes a rearrangement hydrogenation reactor 11, a gas-liquid separator 12, an extraction column 13, a first distillation separator 14, and a second distillation separator 15.

The production apparatus 1 further includes: a flow path L1 for transferring a first fluid containing furfuryl alcohol, hydrogen, and water to the rearrangement hydrogenation reactor 11; a flow path L2 for transferring a second fluid recovered from an outlet of the rearrangement hydrogenation reactor 11 to the gas-liquid separator 12; a flow path L3 for transferring a gas phase separated in the gas-liquid separator 12; a flow path L4 for transferring a liquid phase (third fluid) separated in the gas-liquid separator 12 to the extraction column 13; a flow path L5 for supplying an extraction solvent (for example, toluene in FIG. 1) to the extraction column 13; a flow path L6 for transferring an aqueous phase separated in the extraction column 13; a flow path L7 for transferring an oil phase (fourth fluid) separated in the extraction column 13 to the first distillation separator 14; a flow path L8 for transferring a part or all of the extraction solvent recovered from a top of the first distillation separator 14 to the flow path L5; a flow path L9 for transferring a fraction (fifth fluid) recovered from a bottom of the first distillation separator 14 to the second distillation separator 15; a flow path L10 for transferring purified cyclopentanone recovered from the second distillation separator 15; and a flow path L11 for transferring by-products recovered from the second distillation separator 15.

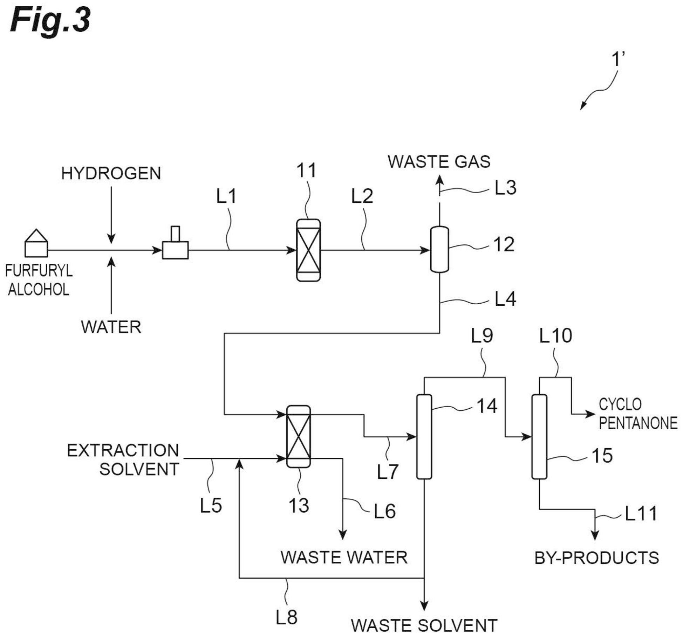

The production apparatus 1 is an example of a production apparatus used when the boiling point of the extraction solvent is lower than that of cyclopentanone. When the boiling point of the extraction solvent is higher than that of cyclopentanone, the flow path L8 may be a flow path for transferring a part or all of the extraction solvent recovered from the bottom of the first distillation separator 14 to the flow path L5, and the flow path L9 may be a flow path for transferring a fraction (fifth fluid) recovered from the top of the first distillation separator 14 to the second distillation separator 15. FIG. 3 is a diagram showing an example of a production apparatus (production apparatus 1') used when the boiling point of the extraction solvent is higher than that of cyclopentanone.

According to the production apparatuses 1 and 1', the above-described production method can be easily carried out.

Although the preferred embodiment of the present disclosure has been described above, the present disclosure is not limited to the above embodiment.

### Examples

Hereinafter, the present disclosure will be described in detail with reference to examples. However, the present disclosure is not limited by these examples.

### <Example 1>

A simulation was performed for the production of cyclopentanone using a production apparatus 1 in FIG. 1. A simulation software "Aspen Plus" manufactured by Aspen Technology, Inc. was used.

Specifically, a simulation was performed for the following process. First, a first fluid prepared by mixing furfuryl alcohol, hydrogen, and water was supplied to the rearrangement hydrogenation reactor 11 through the flow path L1 to obtain a second fluid in which a part of the furfuryl alcohol was converted into cyclopentanone. The second fluid was supplied to a gas-liquid separator 12 through the flow path L2, and was separated into a gas phase and a third fluid composed of a liquid phase. The third fluid was supplied to the extraction column 13 through the flow path L4 and mixed with an extraction solvent (toluene) supplied through the flow path L5. After the two streams were stirred in the extraction column 13, they were separated into an oil phase (fourth fluid) containing toluene and cyclopentanone, and an aqueous phase containing water. The fourth fluid was supplied to the first distillation separator 14 through the flow path L7, and was separated into a fraction mainly composed of toluene and a fifth fluid mainly composed of cyclopentanone. The fraction mainly composed of toluene was mixed with the extraction solvent through the flow path L8 and recycled to the extraction column 13. The fifth fluid was supplied to the second distillation separator 15 through the flow path L9, and was separated into purified cyclopentanone with an increased concentration of cyclopentanone and by-products.

Reaction conditions of the rearrangement hydrogenation reactor and operating conditions of the gas-liquid separator and the extraction column were as shown below, and compositions of fluids passing through each flow path are shown in Table 1.

### (Rearrangement hydrogenation reactor)

Reaction pressure: 5.8 MPaG
Reaction temperature: 200°C
Hydrogen/oxygen-containing organic component: 6.3 (molar ratio)
WHSV: 0.023 h⁻¹
Water:furfuryl alcohol = 20:1 (mass ratio)
Catalyst: 0.04% Pd/SiO₂

### (Gas-liquid separator)

Pressure: 2.0 MPaG
Temperature: 40°C

### (Extraction column)

Pressure: 0.5 MPaG
Temperature: 50°C
Number of stages: 10, countercurrent flow

### <Comparative Example 1>

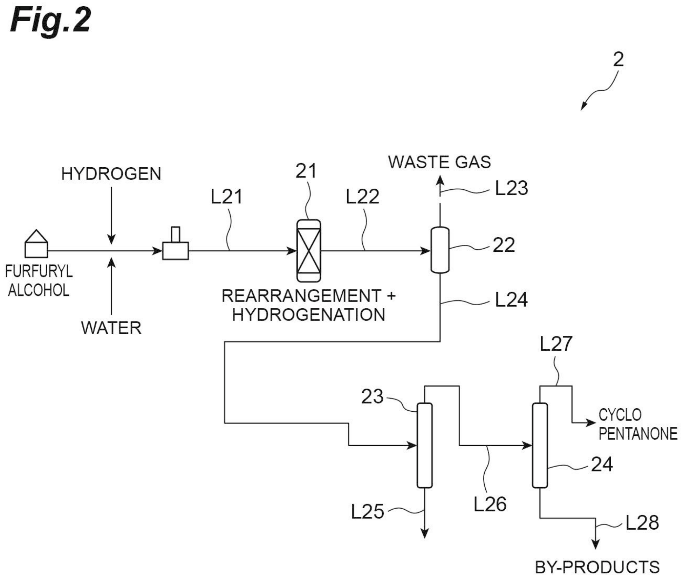

A simulation was performed for the production of cyclopentanone using a production apparatus 2 in FIG. 2. A simulation software "Aspen Plus" manufactured by Aspen Technology, Inc. was used.

A production apparatus 2 in FIG. 2 includes a rearrangement hydrogenation reactor 21, a gas-liquid separator 22, a first distillation separator 23, and a second distillation separator 24. The production apparatus 2 further includes: a flow path L21 for transferring a first fluid containing furfuryl alcohol, hydrogen, and water to the rearrangement hydrogenation reactor 21; a flow path L22 for transferring a second fluid recovered from an outlet of the rearrangement hydrogenation reactor 21 to the gas-liquid separator 22; a flow path L23 for transferring a gas phase separated in the gas-liquid separator 22; a flow path L24 for transferring a liquid phase (third fluid) separated in the gas-liquid separator 22 to the first distillation separator 23; a flow path L25 for removing a fluid containing water from the first distillation separator 23 to outside of the system; a flow path L26 for transferring a fraction containing cyclopentanone from the first distillation separator 23 to the second distillation separator; a flow path L27 for transferring purified cyclopentanone recovered from the second distillation separator; and a flow path L28 for transferring by-products recovered from the second distillation separator.

Specifically, a simulation was performed for the following process. First, a first fluid prepared by mixing furfuryl alcohol, hydrogen, and water was supplied to the rearrangement hydrogenation reactor 21 through the flow path L21 to obtain a second fluid in which a part of the furfuryl alcohol was converted into cyclopentanone. The second fluid was supplied to a gas-liquid separator 22 through the flow path L22, and was separated into a gas phase and a third fluid composed of a liquid phase. The third fluid was supplied to the first distillation separator 23 through the flow path L24, and was separated into a fraction containing cyclopentanone and wastewater with an increased concentration of water. The fraction containing cyclopentanone was supplied to the second distillation separator 24 through the flow path L26, and was separated into purified cyclopentanone with an increased concentration of cyclopentanone and by-products.

Reaction conditions of the rearrangement hydrogenation reactor and operating conditions of the gas-liquid separator were the same as those in Example 1, and compositions of fluids passing through each flow path are shown in Table 2.

### <Comparative Example 2>

A simulation was performed for a production process similar to that in Example 1, except that furfural was used instead of furfuryl alcohol. Compositions of fluids passing through each flow path are shown in Table 3.

### <Example 2>

A simulation was performed for the production of cyclopentanone using a production apparatus 1' in FIG. 3. A simulation software "Aspen Plus" manufactured by Aspen Technology, Inc. was used.

Specifically, a simulation was performed for the following process. First, a first fluid prepared by mixing furfuryl alcohol, hydrogen, and water was supplied to the rearrangement hydrogenation reactor 11 through the flow path L1 to obtain a second fluid in which a part of the furfuryl alcohol was converted into cyclopentanone. The second fluid was supplied to a gas-liquid separator 12 through the flow path L2, and was separated into a gas phase and a third fluid composed of a liquid phase. The third fluid was supplied to the extraction column 13 through the flow path L4 and mixed with an extraction solvent (1,2,3,5-tetramethylbenzene) supplied through the flow path L5. After the two streams were stirred in the extraction column 13, they were separated into an oil phase (fourth fluid) containing 1,2,3,5-tetramethylbenzene and cyclopentanone, and an aqueous phase containing water. The fourth fluid was supplied to the first distillation separator 14 through the flow path L7, and was separated into a fraction mainly composed of 1,2,3,5-tetramethylbenzene and a fifth fluid mainly composed of cyclopentanone. The fraction mainly composed of 1,2,3,5-tetramethylbenzene was mixed with the extraction solvent through the flow path L8 and recycled to the extraction column 13. The fifth fluid was supplied to the second distillation separator 15 through the flow path L9, and was separated into purified cyclopentanone with an increased concentration of cyclopentanone and by-products.

Reaction conditions of the rearrangement hydrogenation reactor and operating conditions of the gas-liquid separator and the extraction column were as shown below, and compositions of fluids passing through each flow path are shown in Table 4.

### (Rearrangement hydrogenation reactor)

Reaction pressure: 5.8 MPaG
Reaction temperature: 200°C
Hydrogen/oxygen-containing organic component: 6.3 (molar ratio)
WHSV: 0.5 h⁻¹
Water: furfuryl alcohol = 10:1 (mass ratio)
Catalyst: 0.04% Pd/SiO₂

### (Gas-liquid separator)

Pressure: 2.0 MPaG
Temperature: 40°C

### (Extraction column)

Pressure: 0.5 MPaG
Temperature: 50°C
Number of stages: 10, countercurrent flow

In Tables 1 to 4, numerical values of each composition represent mass ratios based on flow rate. In addition, in Tables 1 to 4, A-1 represents furfuryl alcohol, A-2 represents furfural, A-3 represents 2-cyclopentenone, and A-4 represents 4-hydroxy-2-cyclopentenone.

**[Table 1]**

| 3 | L1 | L2 | L3 | L4 | L5 | L6 | L7 | L8 | L9 | L10 | L11 |
|---|---|---|---|---|---|---|---|---|---|---|---|
| Flow rate [kg/h] | 100 | 100 | 0.50 | 99.50 | 4.76 | 96.13 | 50.27 | 42.14 | 3.42 | 3.12 | 0.30 |
| Hydrogen | 0.64 | 0.50 | 96.85 | 0.01 | 0.00 | 0.00 | 0.03 | 0.00 | 0.00 | 0.00 | 0.00 |
| Water | 94.39 | 95.18 | 3.10 | 95.64 | 0.00 | 98.99 | 0.04 | 0.04 | 0.00 | 0.00 | 0.00 |
| Toluene | 0.00 | 0.00 | 0.00 | 0.00 | 100.00 | 0.07 | 93.10 | 99.94 | 0.08 | 0.08 | 0.00 |
| Cyclopentanone | 0.00 | 3.27 | 0.05 | 3.29 | 0.00 | 0.13 | 6.28 | 0.02 | 91.93 | 98.91 | 19.65 |
| A-1 | 4.97 | 0.04 | 0.00 | 0.04 | 0.00 | 0.04 | 0.01 | 0.00 | 0.16 | 0.00 | 1.79 |
| A-2 | 0.00 | 0.19 | 0.00 | 0.20 | 0.00 | 0.00 | 0.39 | 0.00 | 5.68 | 0.25 | 62.03 |
| A-3 | 0.00 | 0.07 | 0.00 | 0.07 | 0.00 | 0.00 | 0.13 | 0.00 | 1.89 | 0.49 | 16.44 |
| A-4 | 0.00 | 0.08 | 0.00 | 0.08 | 0.00 | 0.07 | 0.02 | 0.00 | 0.26 | 0.27 | 0.09 |
| Others | 0.00 | 0.67 | 0.00 | 0.67 | 0.00 | 0.70 | 0.00 | 0.00 | 0.00 | 0.00 | 0.00 |

**[Table 2]**

| | L21 | L22 | L23 | L24 | L25 | L26 | L27 | L28 |
|---|---|---|---|---|---|---|---|---|
| Flow rate [kg/h] | 100 | 100 | 0.56 | 99.44 | 91.51 | 7.93 | 3.20 | 4.73 |
| Hydrogen | 0.64 | 0.50 | 89.11 | 0.00 | 0.00 | 0.05 | 0.13 | 0.00 |
| Water | 94.39 | 95.18 | 10.72 | 95.65 | 99.22 | 54.42 | 53.86 | 54.80 |
| Toluene | 0.00 | 0.00 | 0.00 | 0.00 | 0.00 | 0.00 | 0.00 | 0.00 |
| Cyclopentanone | 0.00 | 3.27 | 0.17 | 3.29 | 0.00 | 41.25 | 41.83 | 40.85 |
| A-1 | 4.97 | 0.04 | 0.00 | 0.04 | 0.05 | 0.00 | 0.00 | 0.01 |
| A-2 | 0.00 | 0.19 | 0.00 | 0.20 | 0.00 | 2.46 | 2.14 | 2.66 |
| A-3 | 0.00 | 0.07 | 0.00 | 0.07 | 0.00 | 0.82 | 2.04 | 0.00 |
| A-4 | 0.00 | 0.08 | 0.00 | 0.08 | 0.00 | 1.00 | 0.00 | 1.68 |
| Others | 0.00 | 0.67 | 0.00 | 0.67 | 0.73 | 0.00 | 0.00 | 0.00 |

**[Table 3]**

| | L1 | L2 | L3 | L4 | L5 | L6 | L7 | L8 | L9 | L10 | L11 |
|---|---|---|---|---|---|---|---|---|---|---|---|
| Flow rate [kg/h] | 100 | 100 | 0.52 | 99.48 | 5.29 | 97.01 | 50.86 | 43.10 | 2.94 | 1.11 | 1.83 |
| Hydrogen | 0.64 | 0.52 | 96.88 | 0.01 | 0.00 | 0.00 | 0.03 | 0.00 | 0.00 | 0.00 | 0.00 |
| Water | 94.39 | 94.65 | 3.10 | 95.13 | 0.00 | 97.55 | 0.04 | 0.04 | 0.00 | 0.00 | 0.00 |
| Toluene | 0.00 | 0.00 | 0.00 | 0.00 | 100 | 0.07 | 94.97 | 99.96 | 14.35 | 38.03 | 0.00 |
| Cyclopentanone | 0.00 | 0.68 | 0.01 | 0.68 | 0.00 | 0.03 | 1.29 | 0.00 | 22.27 | 58.99 | 0.02 |
| A-1 | 0.00 | 0.95 | 0.00 | 0.96 | 0.00 | 0.85 | 0.26 | 0.00 | 4.39 | 0.00 | 7.04 |
| A-2 | 4.97 | 1.49 | 0.01 | 1.50 | 0.00 | 0.00 | 2.93 | 0.00 | 50.60 | 0.25 | 81.11 |
| A-3 | 0.00 | 0.17 | 0.00 | 0.17 | 0.00 | 0.00 | 0.32 | 0.00 | 5.61 | 0.27 | 8.85 |
| A-4 | 0.00 | 0.25 | 0.00 | 0.25 | 0.00 | 0.23 | 0.05 | 0.00 | 0.93 | 2.46 | 0.01 |
| Others | 0.00 | 1.29 | 0.00 | 1.30 | 0.00 | 1.27 | 0.11 | 0.00 | 1.85 | 0.00 | 2.97 |

**[Table 4]**

| | L1 | L2 | L3 | L4 | L5 | L6 | L7 | L8 | L9 | L10 | L11 |
|---|---|---|---|---|---|---|---|---|---|---|---|
| Flow rate [kg/h] | 100 | 100 | 34.67 | 65.33 | 3.38 | 60.84 | 37.65 | 29.78 | 4.54 | 4.15 | 0.39 |
| Hydrogen | 33.76 | 33.58 | 96.83 | 0.01 | 0.00 | 0.00 | 0.03 | 0.00 | 0.00 | 0.00 | 0.00 |
| Water | 59.61 | 60.66 | 3.07 | 91.22 | 0.00 | 97.95 | 0.03 | 0.04 | 0.00 | 0.00 | 0.00 |
| Tetramethylbenzene | 0.00 | 0.00 | 0.00 | 0.00 | 100 | 0.08 | 87.91 | 99.96 | 0.31 | 0.34 | 0.02 |
| Cyclopentanone | 0.00 | 4.36 | 0.10 | 6.63 | 0.00 | 0.27 | 11.07 | 0.01 | 91.68 | 98.48 | 18.81 |
| A-1 | 6.62 | 0.05 | 0.00 | 0.08 | 0.00 | 0.08 | 0.02 | 0.00 | 0.16 | 0.00 | 1.85 |
| A-2 | 0.00 | 0.26 | 0.00 | 0.40 | 0.00 | 0.00 | 0.69 | 0.00 | 5.70 | 0.32 | 63.29 |
| A-3 | 0.00 | 0.09 | 0.00 | 0.13 | 0.00 | 0.00 | 0.23 | 0.00 | 1.89 | 0.58 | 15.95 |
| A-4 | 0.00 | 0.11 | 0.00 | 0.16 | 0.00 | 0.15 | 0.03 | 0.00 | 0.26 | 0.27 | 0.08 |
| Others | 0.00 | 0.89 | 0.00 | 1.37 | 0.00 | 1.47 | 0.00 | 0.00 | 0.00 | 0.00 | 0.00 |

### Reference Signs List

1, 2 Production apparatus, 11, 21 Rearrangement hydrogenation reactor, 12, 22 Gas-liquid separator, 13 Extraction column, 14, 23 First distillation separator, 15, 24 Second distillation separator

## Claims

1. A production method for cyclopentanone comprising:
a rearrangement hydrogenation step of bringing a first fluid containing furfuryl alcohol, water, and hydrogen into contact with a hydrogenation catalyst to obtain a reaction product containing cyclopentanone and water;
an extraction step of extracting cyclopentanone from the reaction product with an extraction solvent to obtain an oil phase containing cyclopentanone; and
a recovery step of recovering cyclopentanone from the oil phase.

2. The production method according to claim 1,
wherein the recovery step includes
a separation step of removing at least a part of the extraction solvent from the oil phase; and
a purification step of purifying cyclopentanone from a residue of the separation step.

3. The production method according to claim 1, wherein the extraction solvent includes at least one selected from the group consisting of aromatic hydrocarbons and alicyclic hydrocarbons.

4. Purified cyclopentanone comprising:
cyclopentanone; and
at least one trace component selected from the group consisting of furfural, 4-hydroxy-2-cyclopentenone, 2-cyclopentenone, toluene, ethylbenzene, ethylcyclohexane, and dimethylcyclohexane,
wherein a content of the cyclopentanone is 98 mass% or more,
a content of the trace component is 0.1 mass% or more and less than 2 mass%, and
a percentage of 4-hydroxy-2-cyclopentenone and furfuryl alcohol in the trace component is less than 50 mass%.
